(19) Europäisches Patentamt
European Patent Office
Office européen des brevets

(11) **EP 3 302 664 B1**

(12) **EUROPEAN PATENT SPECIFICATION**

(45) Date of publication and mention
of the grant of the patent:
**05.08.2020 Bulletin 2020/32**

(51) Int Cl.:
***A61M 16/00*** *(2006.01)*

(21) Application number: **16728111.2**

(22) Date of filing: **01.06.2016**

(86) International application number:
**PCT/IB2016/053206**

(87) International publication number:
**WO 2016/193915 (08.12.2016 Gazette 2016/49)**

(54) **NON-INVASIVE METHOD FOR MONITORING PATIENT RESPIRATORY STATUS VIA SUCCESSIVE PARAMETER ESTIMATION**

NICHTINVASIVES VERFAHREN ZUR ÜBERWACHUNG DES ATEMSTATUS EINES PATIENTEN MITTELS AUFEINANDERFOLGENDER PARAMETERSCHÄTZUNG

PROCÉDÉ NON INVASIF DE SURVEILLANCE DE L'ÉTAT RESPIRATOIRE D'UN PATIENT PAR L'INTERMÉDIAIRE D'UNE ESTIMATION DE PARAMÈTRES SUCCESSIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.06.2015 US 201562169863 P**

(43) Date of publication of application:
**11.04.2018 Bulletin 2018/15**

(73) Proprietor: **Koninklijke Philips N.V.**
**5656 AG Eindhoven (NL)**

(72) Inventors:
• **WANG, Dong**
**5656 AE Eindhoven (NL)**
• **VICARIO, Francesco**
**5656 AE Eindhoven (NL)**
• **ALBANESE, Antonio**
**5656 AE Eindhoven (NL)**
• **KARAMOLEGKOS, Nikolaos**
**5656 AE Eindhoven (NL)**
• **CHBAT, Nicolas Wadih**
**5656 AE Eindhoven (NL)**

(74) Representative: **de Haan, Poul Erik**
**Philips International B.V.**
**Philips Intellectual Property & Standards**
**High Tech Campus 5**
**5656 AE Eindhoven (NL)**

(56) References cited:
**EP-A1- 0 904 730          WO-A2-2014/207623**
**US-A1- 2003 010 339     US-A1- 2004 040 560**
**US-A1- 2015 073 291**

**Description**

<u>FIELD</u>

The following relates generally to systems and methods for monitoring and characterizing respiratory parameters during patient ventilation. It finds particular application in a system to provide real-time diagnostic information to a clinician to personalize a patient's ventilation strategy and improve patient outcomes and will be described with particular reference thereto. However, it is to be understood that it also finds application in other usage scenarios and is not necessarily limited to the aforementioned application.

<u>BACKGROUND</u>

Real-time assessment of the respiratory system's parameters (resistance $R_{rs}$ and compliance $C_{rs}$) and patient's inspiratory effort (respiratory muscle pressure $P_{mus}(t)$) provides invaluable diagnostic information for clinicians to optimize ventilation therapy.

A good $P_{mus}(t)$ estimation can be used to quantify patient's inspiratory effort and select the appropriate level of ventilation support in order to avoid respiratory muscle atrophy and fatigue. Moreover, the estimated $P_{mus}(t)$ waveform can also be used for triggering and cycling off the ventilator so as to reduce patient-ventilator dyssynchrony. Estimates of $R_{rs}$ and $C_{rs}$ are also important, as they provide quantitative information to clinicians about the mechanical properties of the patient's respiratory system and they can be used to diagnose respiratory diseases and better select the appropriate ventilator settings.

$P_{mus}(t)$ is traditionally estimated via esophageal pressure measurement. This technique is invasive, in the sense that a balloon needs to be inserted inside the patient's esophagus, and moreover, not reliable when applied during long periods in intensive care conditions.

Another option to estimate $P_{mus}(t)$ is to calculate it based on the Equation of Motion of the Lungs. Assuming $R_{rs}$ and $C_{rs}$ are known, it is indeed possible to estimate $P_{mus}(t)$ via the following equation, known as the Equation of Motion of the lungs:

$$P_y(t) = R_{rs}\dot{V}(t) + \left(\frac{1}{C_{rs}}\right)V(t) + P_{mus}(t) + P_0 \qquad (1)$$

where $P_y(t)$ is the pressure measured at the Y-piece of the ventilator, $\dot{V}(t)$ is the flow of air into and out of the patient's respiratory system (measured again at the Y-piece), $V(t)$ is the net volume of air delivered by the ventilator to the patient (measured by integrating the flow signal $\dot{V}(t)$ over time), $P_0$ is a constant term to account for the pressure at the end of expiration (needed to balance the equation but not interesting per se) and will be considered as part of $P_{mus}(t)$ in the following discussion. However, $R_{rs}$ and $C_{rs}$ have to be measured or estimated first.

$R_{rs}$ and $C_{rs}$ may be estimated by applying the flow-interrupter technique (also called End Inspiratory Pause, EIP), which however interferes with the normal operation of the ventilator, or under specific conditions where the term $P_{mus}(t)$ can be "reasonably" assumed to be zero (i.e. totally unload patient's respiratory muscles). These conditions include: periodic paralysis in which the patient is under Continuous Mandatory Ventilation (CMV); periodic high pressure support (PSV) level; specific portions of each PSV breath that extend both during the inhalation and the exhalation phases; and exhalation portions of PSV breaths where the flow signal satisfies specific conditions that are indicative of the absence of patient's inspiratory effort.

$R_{rs}$ and $C_{rs}$ estimation using the EIP maneuver has certain drawbacks and relies on certain assumptions. The EIP maneuver interrupts the normal ventilation needed by the patient. It also assumes that patient respiratory muscles are fully relaxed during the EIP maneuver in order for the $R_{rs}$ and $C_{rs}$ computation to be valid. Further, the $R_{rs}$ and $C_{rs}$ estimates obtained via the EIP maneuver, which affect the estimate of $P_{mus}(t)$ on the following breath, are assumed to be constant until the next EIP maneuver is executed, so that continuous and real-time estimates of $R_{rs}$ and $C_{rs}$ are not obtained. In practice, changes in patient's conditions can occur in between two consecutive EIP maneuvers, and this would jeopardize the estimate of $P_{mus}(t)$. A further disadvantage is that the static maneuver (EIP) is performed in a specific ventilation mode (Volume Assisted Control, VAC) and the obtained values for R and C might not be representative of the true values that govern the dynamics of the lungs in other ventilation modes, such as Pressure Support Ventilation (PSV). Therefore, the accuracy of $P_{mus}(t)$ computed via equation (1) during PSV operation can be compromised.

The above mentioned estimation methods operate on the assumption that $P_{mus}(t)$ is negligible. Implementation of this assumption can be problematic in clinical settings. For example, imposing periodic paralysis and CMV on a patient is generally not clinically feasible. Similarly, imposing periodic high PSV interferes with the normal operation of the ventilator and may not be beneficial to the patient. The assumption of negligible $P_{mus}(t)$ during PSV breaths is debatable, especially during the inhalation phase. Approaches which operate on a chosen portion of the respiration cycle also limit the fraction of data points that are used in the fitting procedure, which makes the estimation results more sensitive to noise.

United States Patent Application Publication No. US 2003/0010339 A1 describes a method and corresponding medical ventilator for nullifying the work of breathing imposed by the ventilation breathing apparatus during ventilation support of a patient.

United States Patent Application Publication No. US 2004/0040560 A1 describes a method of creating a non-invasive predictor of both physiologic and imposed patient effort.

European Patent Application Publication Number EP 0904730 A1 describes a method and apparatus for determining airway resistance and lung compliance using an electrical circuit model in which at least one component parameter is non-linear.

International Patent Application Publication Number WO 2014/207623 A2 presents a non-invasive estimation of intra-pleural pressure.

In the following, non-invasive methods are disclosed for monitoring patient respiratory status via successive parameter estimation, which overcome various foregoing deficiencies and others.

## SUMMARY

According to an aspect of the invention, there is provided a medical ventilator device according to claim 1.

In accordance with one embodiment, a medical ventilator device is described. The device includes a ventilator configured to deliver ventilation to a ventilated patient, a pressure sensor configured to measure the airway pressure $P_y(t)$ at a Y-piece of the ventilator and an air flow sensor configured to measure the air flow $\dot{V}(t)$ into and out of the ventilated patient at the Y-piece of the ventilator. The device also comprises a respiratory system monitor comprising a microprocessor configured to estimate respiratory parameters of the ventilated patient using moving window least squares (MWLS) estimation including (i) respiratory system elastance or compliance ($E_{rs}$ or $C_{rs}$), (ii) respiratory system resistance ($R_{rs}$), and (iii) respiratory muscle pressure ($P_{mus}(t)$).

According to an aspect of the invention, there is provided a method according to claim 14.

In accordance with one embodiment, a method comprises: ventilating a patient using a ventilator; during the ventilating, measuring airway pressure $P_y(t)$ and air flow $\dot{V}(t)$ of air into and out of the patient; using a microprocessor, applying moving window least squares (MWLS) estimation to estimate (i) the patient's respiratory system elastance or compliance $E_{rs}$ or $C_{rs}$, (ii) the patient's respiratory system resistance $R_{rs}$, and (iii) the patient's respiratory muscle pressure $P_{mus}(t)$; and displaying on a display one or more of the respiratory parameters of the patient estimated by applying MWLS estimation.

One advantage resides in providing a non-invasive method for monitoring patient respiratory status via successive parameter estimation including resistance, compliance, and respiratory muscle pressure.

Another advantage resides in providing a ventilator with improved data analysis.

Still further advantages of the present invention will be appreciated to those of ordinary skill in the art upon reading and understand the following detailed description. It is to be appreciated that none, one, two, or more of these advantages may be achieved by a particular embodiment.

## BRIEF DESCRIPTION OF THE DRAWINGS

The disclosure may take form in various components and arrangements of components, and in various steps and arrangement of steps. The drawings are only for purposes of illustrating the preferred embodiments and are not to be construed as limiting the invention.

FIGURE 1 illustrates a ventilation system for use on a patient with the proposed ventilation estimation scheme.
FIGURE 2 illustrates a block diagram of the described estimation scheme.
FIGURE 3 illustrates a moving window least squares algorithm for the $E_{rs}$ estimation.
FIGURE 4 illustrates a moving window least squares algorithm example of the polynomial order of the local $P_{mus}(t)$ waveform.
FIGURE 5 illustrates the maximum ratio combination for the MWLS $R_{rs}$ estimation results.

## DETAILED DESCRIPTION

The following relates to characterization of respiratory parameters during patient ventilation and in particular to the respiratory muscle pressure $P_{mus}(t)$, respiratory resistance $R_{rs}$, and respiratory compliance $C_{rs}$ or elastance $E_{rs}$ = $1/C_{rs}$. In principle, these parameters can be estimated using the Equation of Motion of the Lungs (Equation (1)), which relates these parameters to the pressure $P_y(t)$ at the ventilator mouthpiece and the air flow $\dot{V}(t)$, along with the air volume in the lungs $V(t) = \int \dot{V}(t)dt$. In practice, because the respiratory muscle pressure $P_{mus}(t)$ varies over time, estimating $P_{mus}(t)$, $R_{rs}$, and $E_{rs}$ jointly using the Equation of Motion of the Lungs is generally underdetermined and cannot be analytically solved. Various approaches to dealing with this include measuring additional information using invasive probes, or creating "special case" circumstances by operations such as interrupting normal breathing. Invasive probes have apparent disadvantages, while techniques that rely upon manipulating normal patient breathing cannot provide continuous monitoring of normal respiration and may be detrimental to the patient.

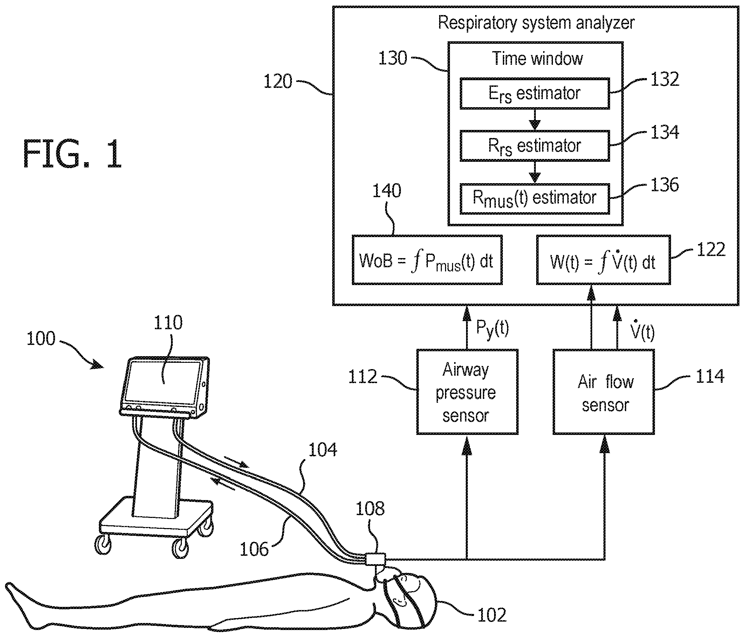

With reference to FIGURE 1, a medical ventilator system includes a medical ventilator **100** that delivers air flow at a positive pressure to a patient **102** via an inlet air hose **104**. Exhaled air returns to the ventilator **100** via an exhalation air hose **106**. A Y-piece **108** of the ventilator system serves to couple air from the discharge end of the inlet air hose **104** to the patient during inhalation and serves to couple exhaled air from the patient into the exhalation air hose **106** during exhalation. Note the Y-piece **108** is sometimes referred to by other nomenclatures, such as a T-piece. Not shown in FIGURE 1 are numerous other ancillary components that may be provided depending upon the respiratory therapy being received by the patient **102**. Such ancillary components may include, by way of illustration: an oxygen bottle or other medical-grade oxygen source for delivering a controlled level of oxygen to the air flow (usually controlled by the Fraction of Inspired Oxygen (FiO$_2$) ventilator parameter set by the physician or other medical personnel); a humidifier plumbed into the inlet line **104**; a nasogastric tube to provide the patient **102** with nourishment; and so forth. The ventilator **100** includes a user interface including, in the illustrative example, a touch-sensitive display component **110** via which the physician, respiratory specialist, or other medical personnel can configure ventilator operation and monitor measured physiological parameters and operating parameters of the ventilator **100**. Additionally or alternatively, the user interface may include physical user input controls (buttons, dials, switches, et cetera), a keyboard, a mouse, audible alarm device(s), indicator light(s), or so forth. It is also noted that the illustrative ventilator **100** is merely an illustrative example.

The illustrative ventilator **100** is a dual-limb ventilator with proximate sensors. However, the disclosed patient respiratory status monitoring techniques may be employed in conjunction with substantially any type of ventilator, such as with a single-limb or dual-limb ventilator, a ventilator having valves or blower, a ventilator with an invasive coupling to the patient (e.g. via a tracheostomy or endotracheal tube) or a ventilator with a noninvasive coupling to the patient (e.g. using a facial mask), a ventilator with proximal sensors for measuring pressure and flow as illustrated or a ventilator without such proximal sensors that relies upon sensors in the ventilator unit, or so forth.

With continuing reference to FIGURE 1, the patient **102** is monitored by various physiological parameter sensors. In particular, FIGURE 1 illustrates two such sensors: an airway pressure sensor **112** that measures pressure $P_y(t)$ at the coupling to the patient (usually measured at the Y-piece **108**, hence $P_y(t)$) and an air flow sensor **114** that measures air flow $\dot{V}(t)$ to or from the patient (also usually measured at the Y-piece **108**). The sensors **112, 114** may be integrated into the Y-piece **108**, interposed on the air lines **104, 106**, or integrated into the ventilator **100**. During mechanical ventilation, other physiological parameters may be monitored by suitable sensors, such as heart rate, respiratory rate, blood pressure, blood oxygenation (e.g. SpO$_2$), respiratory gases composition (e.g. a capnograph measuring CO$_2$ in respiratory gases), and so forth. Other physiological parameters may be derived from directly measured physiological parameters.

The system further includes a respiratory system analyzer **120** comprising a microprocessor, microcontroller, or other electronic data processing device programmed to process input data including the airway pressure $P_y(t)$ and air flow $\dot{V}(t)$ to generate information about the patient respiratory system parameters: resistance $R_{rs}$, compliance $C_{rs}$ (or, equivalently, elastance $E_{rs}=1/C_{rs}$), and the patient's inspiratory effort characterized as a function of time by the respiratory muscle pressure $P_{mus}(t)$. These parameters are determined as a function of time, in real-time, by evaluating the Equation of Motion of the Lungs (Equation (1)) using moving window least squares estimation (MWLS) applied to the airway pressure $P_y(t)$ and air flow $\dot{V}(t)$ along with the air volume $V(t) = \int \dot{V}(t)dt$ determined from $\dot{V}(t)$ by an air flow integrator **122**. (Alternatively, a dedicated air volume sensor may be employed). To overcome the underdetermined nature of Equation (1), the MWLS estimation is performed using successive estimation of: (1) the elastance or compliance ($E_{rs}$ or $C_{rs}$) parameter via an $E_{rs}$ estimator **132**; followed by (2) estimation of the resistance ($R_{rs}$) parameter via an $R_{rs}$ estimator **134**; followed by (3) estimation of the respiratory muscle pressure ($P_{mus}(t)$) parameter via a $P_{mus}(t)$ estimator **136**.

These successive estimators **132, 134, 136** are applied within the time window **130** which is generally of duration two seconds or less, and more preferably of duration one second or less, and in an illustrative example of duration 0.6 seconds with data sampling at 100 Hz so that the time window contains 60 samples. An upper limit on the duration of the time window is imposed by the respiration rate, which for a normal adult is typically 12 to 20 breaths per minute corresponding to a breathing cycle of duration 3-5 seconds. The duration of the time window **130** is preferably a fraction of the breathing cycle duration so that the parameters $E_{rs}$ and $R_{rs}$ can be reasonably assumed to be constant within each time window **130**, and variation of $P_{mus}(t)$ within each time window **130** can be represented using a relatively simple approximation function (e.g. a low-order polynomial in the illustrative examples disclosed herein).

The estimators **132, 134, 136** are successively applied within each time window **130**, and for each successive (and partially overlapping) time interval **130** (hence the term "moving" time window), to provide estimation of $E_{rs}$, $R_{rs}$, and $P_{mus}(t)$ in real time. In the illustrative examples, the values of $E_{rs}$ and $R_{rs}$ are assumed to be constant within each time window **130,** so that the estimation of these parameters is in real-time with a time resolution comparable to the duration of the time window **130,** e.g. two second or less in some embodiments, or more preferably one second or less, and 0.6 seconds in the illustrative examples. If successive time windows partially overlap, this can further improve the effective time resolution. The real-time estimation of $P_{mus}(t)$ can be of higher temporal resolution than $E_{rs}$ and $R_{rs}$, since variation of $P_{mus}(t)$ with time within the time window **130** is, in the illustrative examples, modeled by a low-order polynomial function of time.

The approach disclosed herein leverages the recognition that, of the three parameters being estimated, the elastance/compliance ($E_{rs}$ or $C_{rs}$) generally varies most slowly over time. In the Equation of Motion of the Lungs (Equation (1)), $E_{rs}$ is the coefficient of the air volume $V(t)$ which, as an integral, varies slowly over time. The next most slowly varying parameter is generally the resistance $R_{rs}$, which is the coefficient of the air flow $\dot{V}(t)$. Finally, the respiratory muscle pressure $P_{mus}(t)$ has the potential to vary most rapidly over time as it changes in response to the patient actively inhaling and exhaling. In view of this, the illustrative examples of the $P_{mus}(t)$ estimator **136** do not assume $P_{mus}(t)$ is a constant within the time window **130,** but instead employ a low-order approximation polynomial function. Instead of a low-order polynomial approximation of $P_{mus}(t)$ within the time window **130,** in other contemplated embodiments some other parameterized function of time is contemplated, such as a spline function.

With continuing reference to FIGURE 1, the outputs $E_{rs}$ (or $C_{rs}$), $R_{rs}$, and $P_{mus}(t)$ can be used for various purposes. In one application, one or more of the estimated parameters may be displayed on the display component **110** of the ventilator **100,** for example as a numeric real-time value and/or as a trend line plotted as a function of time. Typically, the respiratory elastance or compliance ($E_{rs}$ or $C_{rs}$) and the respiratory resistance ($R_{rs}$) are of most interest to the clinician and are suitably displayed and/or trended. The respiratory muscle pressure $P_{mus}(t)$ is a waveform acquired as a function of time in real-time during normal clinically operative mechanical ventilation - accordingly, $P_{mus}(t)$ can be used by the ventilator **100** for triggering and cycling off the mechanical ventilation so as to reduce patient-ventilator dyssynchrony (that is, to synchronize application of positive pressure by the ventilator **100** with the inhalation portion of the patient's respiratory muscle action).

In some embodiments, a work of breathing (WoB) estimator **140** integrates the respiratory muscle pressure $P_{mus}(t)$ over volume, i.e. $WoB = \int P_{mus}(t) dV(t)$. The WoB is a metric of how much effort the patient **102** is applying to breathe on his or her own. The WoB may be displayed and/or trended on the display component **110** to provide the clinician with useful information for setting ventilator pressure settings in ventilation modes such as Pressure Support Ventilation (PSV). Moreover, since the WoB estimator **140** provides WoB in real-time (e.g. with a time lag and resolution on the order of a second or less in some embodiments) the ventilator **100** optionally employs the WoB as a feedback control parameter, e.g. adjusting controlled ventilator settings to maintain the WoB at a constant set-point value. For example, if the WoB increases, this implies the patient **102** is struggling to breathe and accordingly the positive pressure applied by the ventilator **100** in PSV mode should be increased to provide the struggling patient with increased respiration assistance.

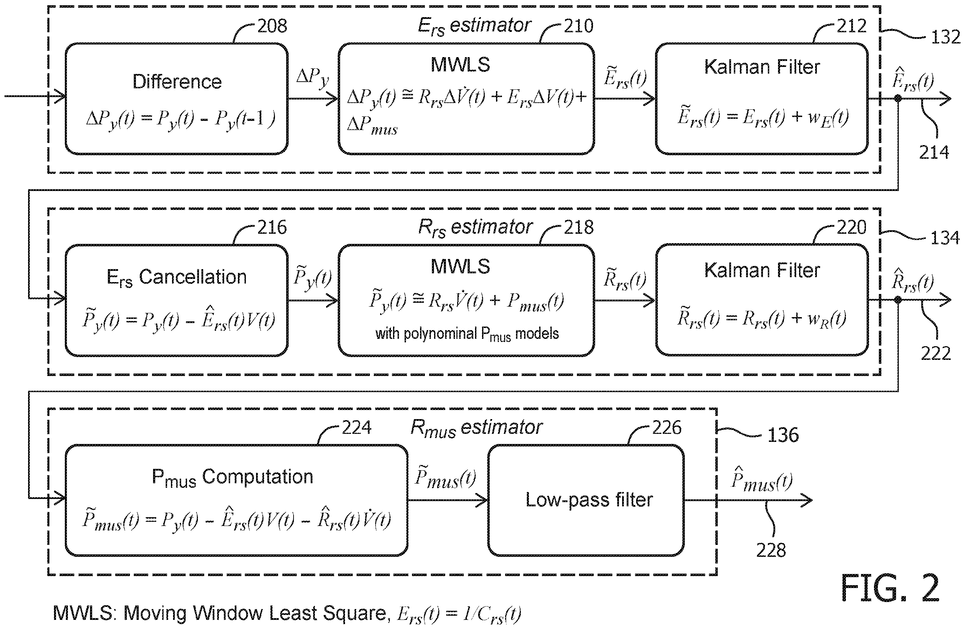

With reference to FIGURE 2, some illustrative embodiments of the successive estimators **132, 134, 136** are described, successive estimation of the parameters $E_{rs}$, $R_{rs}$, and $P_{mus}(t)$ over a time window of a fraction of a second over which the parameters $E_{rs}$ and $R_{rs}$ are assumed to be constant is shown. In the first pass (performed by the $E_{rs}$ estimator **132**), all three parameters $E_{rs}$, $R_{rs}$, and $\Delta P_{mus}(t)$ are assumed to be constant over the time window **130** and are computed simultaneously - but only the estimated $\hat{E}_{rs}$ is retained from this first pass. (In notation used herein, the overscript "hat", i.e. $\hat{p}$, is used to indicate the estimated value of parameter $p$.) In a second pass (performed by the $R_{rs}$ estimator **134**), the contribution of the now known (estimated) $\hat{E}_{rs}$ is removed by subtraction, and the remaining portion of the Equation of Lung Motion is fitted for $R_{rs}$ and $P_{mus}(t)$, the latter being approximated using a low order polynomial (n=0, 1, or 2). In experiments, it was found that the best choice of polynomial order is dependent upon the respiratory phase at which the time window **130** is located due to possible overfitting - as respiratory phase is not known a priori, in illustrative embodiments disclosed herein a weighted combination of polynomials of zeroeth, first, and second order is used. The output of the $R_{rs}$ estimator **134** is the estimated value of the respiratory resistance, i.e. $\hat{R}_{rs}$. Finally, in a third pass (performed by the $P_{mus}(t)$ estimator **136**), the contribution of the now known (estimated) $\hat{R}_{rs}$ is removed by further subtraction, and the remaining portion of the Equation of Lung Motion is directly fitted to obtain the estimated respiratory muscle pressure, i.e. $\hat{P}_{mus}(t)$.

With continuing reference to FIGURE 2, the illustrative $E_{rs}$ estimator **132** is further described. At **208** a difference operation is performed on the airway pressure $P_y(t)$ and the output $\Delta P_y(t)$ is calculated as $\Delta P_y(t) = P_y(t) - P_y(t - 1)$. A Moving Window Least Squares (MWLS) estimator is used to at **210** to continuously estimate $E_{rs}(t)$ - which is the respiratory system's elastance, $E_{rs}(t) = 1/C_{rs}(t)$ - and is based on the following difference equation:

$$\Delta P_y(t) \cong R_{rs}\Delta\dot{V}(t) + E_{rs}\Delta V(t) + \Delta P_{mus}$$

It should be noted that $E_{rs}(t)$ is estimated as a function of time insofar as the estimate $\hat{E}_{rs}$ is generated for each time window **130,** so that the time function $\hat{E}_{rs}(t)$ is generated as the value $\hat{E}_{rs}$ for successive time windows **130** as successive (partially overlapping) time windows are applied over time. However inside each time window **130,** $\Delta P_{mus}(t)$, the difference signal of the $P_{mus}(t)$ waveform, and the parameters $R_{rs}(t)$ and $E_{rs}(t)$ are modeled as constants and jointly estimated by a least squares minimization method. For the $E_{rs}$ estimator **132,** only the estimate of $E_{rs}(t)$, namely $\hat{E}_{rs}$, is used (after filtering by a Kalman filter **212** in the illustrative example of FIGURE 2), while the other estimation outputs are discarded. Moreover, the $E_{rs}$ estimator **132** also calculates the variance of the estimate $\hat{E}_{rs}$, denoted herein as $\delta_{\hat{E}_{rs}}$.

The input to the MWLS estimator **210** is the difference signal of $P_y(t)$, that is, $\Delta P_y(t)$, which is output by the difference operation **208.** Based on the equation of the motion (Equation (1)), $\Delta P_y(t)$ can be modeled as:

$$\Delta P_y(t) \cong R_{rs}(t)\Delta \dot{V}(t) + E_{rs}(t)\Delta V(t) + \Delta P_{mus}(t)$$

where $\Delta \dot{V}(t) = \dot{V}(t) - \dot{V}(t-1)$ is the flow difference signal, $\Delta V(t) = V(t) - V(t-1) = \dot{V}(t)T$ is the volume difference signal (where T is the sampling time interval, e.g. sampling at 100 Hz corresponds to T=0.01 sec), and $\Delta P_{mus}(t) = P_{mus}(t) - P_{mus}(t-1)$ is the $P_{mus}(t)$ difference signal.

In the following, the size (or duration) of the sliding time window **130** is denoted as L, which is optionally a system parameter that can be set by the user. The sliding window at a current time t spans the interval [t-L+1, t]. For the MWLS estimator **210,** the $P_{mus}(t)$ difference signal, $\Delta P_{mus}(t)$, in the sliding window is modeled as a constant, $\Delta P_{mus}$. It is further assumed that $R_{rs}$ and $E_{rs}$ are constant in the sliding time window **130.** Therefore, the equation for $\Delta P_y(t)$ becomes:

$$\Delta P_y(t) \cong R_{rs}\Delta \dot{V}(t) + E_{rs}\Delta V(t) + \Delta P_{mus}$$

At time t, the MWLS algorithm **210** uses the input signals in the sliding window **130,** that is to say, the samples $\Delta P_y(n)$ and $\dot{V}(n)$ in the interval $t - L + 1 \leq n \leq t$, to estimate $R_{rs}$, $E_{rs}$, and $\Delta P_{mus}$ jointly, but only the $E_{rs}$ estimate $\hat{E}_{rs}$ is used in the subsequent operations (i.e. the subsequent estimators **134, 136**).

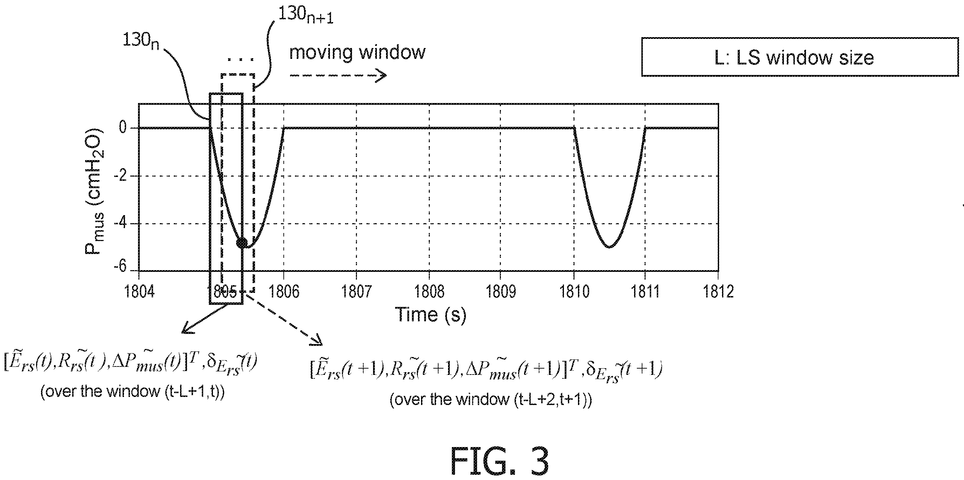

As further shown in FIGURE 3, specifically, at time t, the MWLS formulation solves the least square problem **300** based on the equation described above:

At time t,

$$\Delta P_y(t) \cong R_{rs}\Delta \dot{V}(t) + E_{rs}\Delta V(t) + \Delta P_{mus}$$

$$[\tilde{E}_{rs}, \tilde{R}_{rs}, \Delta \tilde{P}_{mus}]^T = (X^T X)^{-1} X^T Y$$

$$Y = \left[\Delta P_y(t), \Delta P_y(t-1), \dots, \Delta P_y(t-L+1)\right]^T$$

$$X = [\mathrm{x}(t), x(t-1), \dots, x(t-L+1)]^T$$

$$x(t) = \left[\Delta V(t), \Delta \dot{V}(t), 1\right]^T$$

$$\delta_{\tilde{E}_{rs}} = (X^T X)^{-1}(1,1) * \delta_{\Delta P_y}$$

Moreover, the variance of the $E_{rs}$ estimate, $\delta_{\tilde{E}_{rs}}$, is also calculated, where $\delta_{\Delta P_y}$ is the least square residual variance,

$$\delta_{\Delta P_y} = \left( Y - X[\tilde{E}_{rs}, \tilde{R}_{rs}, \Delta \tilde{P}_{mus}]^T \right)^T ( Y - X[\tilde{E}_{rs}, \tilde{R}_{rs}, \Delta \tilde{P}_{mus}]^T )/L$$

As indicated in FIGURE 3, the MWLS estimation **210** is performed continuously as the moving window moves forward, e.g. window **310n** succeeded by next window **310_{0+1}**, and so forth. Since the MWLS method is sensitive to the $P_y$ measurement noise and the modelling error, only the estimate of $E_{rs}$, $\tilde{E}_{rs}$, is retained by the $E_{rs}$ estimator **132** and

the other estimate outputs (e.g. $\tilde{R}_{rs}$ and $\Delta\hat{P}_{mus}$) are discarded.

To further improve the $E_{rs}$ estimation performance, a Kalman filter 212 is optionally used to reduce the $E_{rs}$ estimation error. As previously mentioned, the respiratory system elastance, $E_{rs}$, typically does not change rapidly as a function of time. The Kalman filter 212 is used to filter the estimation noise in $\tilde{E}_{rs}(t)$ and improve the $E_{rs}(t)$ estimation results. The inputs to the Kalman filter 212 are $\tilde{E}_{rs}(t)$ and $\delta_{\tilde{E}_{rs}}(t)$. The output of the Kalman filter 214 is the final estimate of $E_{rs}(t)$, notated herein as $\hat{E}_{rs}(t)$, and $\hat{E}_{rs}(t) = E_{rs}(t) + \omega_E(t)$ where $\omega_E(t)$ is a noise or uncertainty metric. The above model assumes that $\tilde{E}_{rs}(t)$ is an unbiased estimate of $E_{rs}(t)$ that has a noise term $\omega_E(t) \sim N(0, \delta_{\tilde{E}_{rs}}(t))$.

The Kalman filter can be designed to reduce the MWLS estimation noise based on the following assumptions: (1) a state process equation where $E_{rs}$ changes slowly and can be modelled as a random walk, i.e. $E_{rs}(t) = E_{rs}(t - 1) + \omega_E(t)$, $\omega_E(t) \sim N(0, \delta_E)$; and (2) an observation equation where the MWLS estimate $\tilde{E}_{rs}(t)$ can be modelled as $\tilde{E}_{rs}(t) = E_{rs}(t) + \omega_{LE}(t)$, $\omega_E(t) \sim N(0, \delta_{\tilde{E}_{rs}}(t))$. A standard Kalman filter can be implemented with $A = 1$, $B = 0$, $Q = \delta_E$, $H = 1$, and $R = \delta_{\tilde{E}_{rs}}(t)$. The Kalman filter has certain advantages, including computationally efficient implementation in the context of a sliding time window, intuitive operation and output of weighted averages. The parameter $\delta_E$ is an algorithm parameter that controls the average window length.

With continuing reference to FIGURES 1 and 2, the final output $\hat{E}_{rs}(t)$ 214 of the $E_{rs}$ estimator 132 is utilized by the succeeding $R_{rs}$ estimator 134 in performing the $R_{rs}(t)$ estimation. To estimate $R_{rs}(t)$, the elastic pressure component $E_{rs}V(t)$ is cancelled out of $P_y(t)$ using $\hat{E}_{rs}(t)$. This $E_{rs}$ cancellation operation 216 can be expressed as:

$$\widetilde{P}_y(t) = P_y(t) - \widehat{E}_{rs}(t)\, V(t)$$

The $E_{rs}$ cancellation 216 removes one unknown ($E_{rs}$) from the Equation of Motion of the Lungs, and thus simplifies the $R_{rs}$ estimation. Assuming the estimation $\hat{E}_{rs}(t)$ output by the $E_{rs}$ estimator 132 is correct and the elastic pressure component is perfectly cancelled, the MWLS operation 218 of the $R_{rs}$ estimator 134 optimizes the equation:

$$\widetilde{P}_y(t) \cong R_{rs}\dot{V}(t) + P_{mus}(t)$$

Using the Moving Window Least Squares (MWLS) estimator 218, the respiratory resistance $R_{rs}$ is estimated.

In the $E_{rs}$ estimator MWLS operation 210 of the $E_{rs}$ estimator 132, the respiratory muscle pressure $P_{mus}(t)$ is indirectly estimated as a linear function of t since the difference of $P_{mus}(t)$, namely $\Delta P_{mus}(t)$, is estimated as a constant value $\Delta P_{mus}$ for each time window. However, it has been found herein that this estimate is unduly coarse in the case of the MWLS operation 218 of the $R_{rs}$ estimator 134, and that significantly improved estimation of the respiratory resistance $R_{rs}$ is provided if the time dependence of the respiratory muscle pressure $P_{mus}(t)$ is adaptively modeled in the MWLS operation 218. In illustrative examples herein, $P_{mus}(t)$ is modeled using a low order polynomial, e.g. of order 0 (constant value), 1 (linear), or 2 (quadratic). The order of the $P_{mus}(t)$ polynomial function, M, can significantly change the estimation performance.

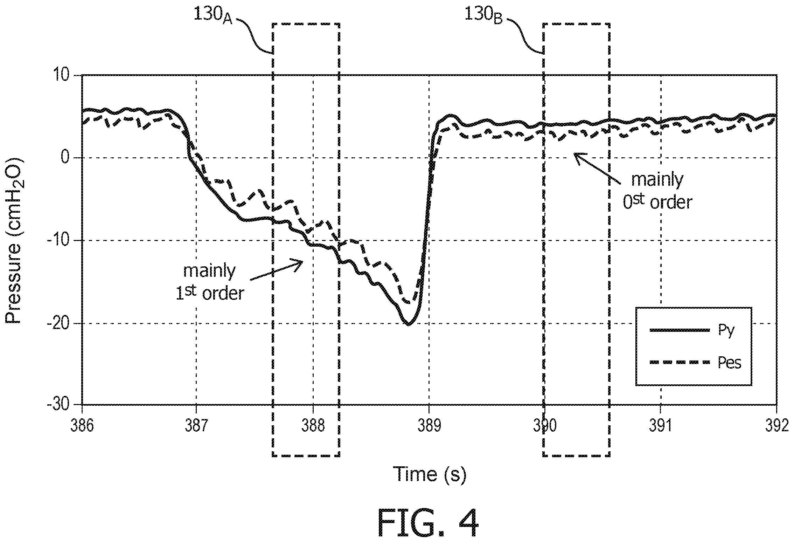

With brief reference to FIGURE 4, moreover, the optimal order M of the polynomial used to model $P_{mus}(t)$ depends on the position of the moving window 130 within the respiratory cycle. In illustrative FIGURE 4, the first time window $130_A$ is located at a respiratory phase for which a first order (M=1) polynomial is an effective model of $P_{mus}(t)$; whereas, for the respiratory phase at which the second time window $130_B$ is located a zeroeth order (M=0) polynomial is effective. However, the respiratory phase at which the current time window 130 resides is generally not an input to the $R_{rs}$ estimator 134.

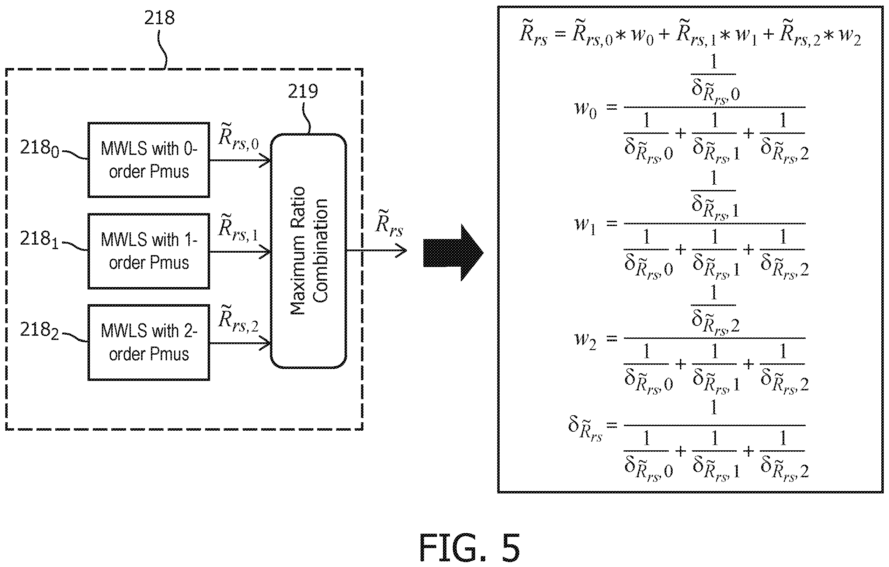

With brief reference to FIGURE 5, for the $R_{rs}$ MWLS 218, the $P_{mus}(t)$ waveform is modeled as an $M^{th}$-order polynomial function (M>=0), i.e. $P_{mus}(t) = a_0 + a_1 t + ... + a_M t^M$, and the $R_{rs}(t)$ parameter is assumed to be constant. (While a polynomial model of $P_{mus}(t)$ is described herein for illustration, other models comprising a parameterized function of time such as a spline model are also contemplated.) To accommodate the differences in optimal polynomial order over the respiratory cycle, the $R_{rs}$ MWLS estimator 218 calculates three $R_{rs}$ estimates: an MWLS estimate $218_0$ using a $0^{th}$- order polynomial (M=0, i.e. $P_{mus}(t)$ is modeled as a constant); an MWLS estimate $218_1$ using a $1^{st}$-order polynomial (M=1, i.e. $P_{mus}(t)$ is modeled as a linear function of t); and an MWLS estimate $218_2$ using $2^{nd}$-order polynomial (M=2, i.e. $P_{mus}(t)$ is modeled as a quadratic function of t). The MWLS formulation for each MWLS estimator $218_0, 218_1, 218_2$ is listed in the righthand box of FIGURE 5 as well as in Table 1 below.

**TABLE 1 - $R_{rs}$ estimator formulations for each $P_{mus}(t)$ model**

| | $P_{mus}$ Model | Moving Window Least Squares Estimator |
|---|---|---|
| 0th Order | $P_{mus}(t) = a_0$ | $\hat{P}_y(t) \cong R_{rs}\dot{V}(t) + P_{mus}(t)$ <br> $[\tilde{R}_{rs,0}, a_0]^T \cong (X^T X)^{-1} X^T Y$ <br> $Y = [\hat{P}_y(t), \hat{P}_y(t-1), \dots, \hat{P}_y(t-L+1)]^T$ <br> $X = [x(t), x(t-1), \dots, x(t-L+1)]^T$ <br> $x(t) = [\dot{V}(t), 1]^T$ <br> $\delta_{\tilde{R}_{rs,0}} = (X^T X)^{-1}(1,1) * \delta_{P_y}$ |
| 1st Order | $P_{mus}(t) = a_0 + a_1 t$ | $\hat{P}_y(t) \cong R_{rs}\dot{V}(t) + P_{mus}(t)$ <br> $[\tilde{R}_{rs,1}, a_0, a_1]^T \cong (X^T X)^{-1} X^T Y$ <br> $Y = [\hat{P}_y(t), \hat{P}_y(t-1), \dots, \hat{P}_y(t-L+1)]^T$ <br> $X = [x(t), x(t-1), \dots, x(t-L+1)]^T$ <br> $x(t) = [\dot{V}(t), 1, t]^T$ <br> $\delta_{\tilde{R}_{rs,1}} = (X^T X)^{-1}(1,1) * \delta_{P_y}$ |
| 2nd Order | $P_{mus}(t) = a_0 + a_1 t + a_2 t^2$ | $\hat{P}_y(t) \cong R_{rs}\dot{V}(t) + P_{mus}(t)$ <br> $[\tilde{R}_{rs,2}, a_0, a_1, a_2]^T \cong (X^T X)^{-1} X^T Y$ <br> $Y = [\hat{P}_y(t), \hat{P}_y(t-1), \dots, \hat{P}_y(t-L+1)]^T$ <br> $X = [x(t), x(t-1), \dots, x(t-L+1)]^T$ <br> $x(t) = [\dot{V}(t), 1, t, t^2]^T$ <br> $\delta_{\tilde{R}_{rs,2}} = (X^T X)^{-1}(1,1) * \delta_{P_y}$ |

With continuing reference to FIGURE 5, the three $R_{rs}(t)$ estimates output by the respective MWLS operations $218_0$, $218_1$, $218_2$ are combined together by a combining operation **219** to produce the final MWLS estimate, $\tilde{R}_{rs}(t)$. The combining operation **219** may use various combinational techniques, such as a maximum ratio combination operation or a minimum variance selection combination. The maximum ratio combination employed by the illustrative combiner **219** assigns the largest weight to the estimate with the least estimation variance (e.g. the one with the best polynomial order) so that the one with the best polynomial order will dominate the $R_{rs}$ estimation output. The MWLS **218** also calculates the variance of $\tilde{R}_{rs}(t)$, $\delta_{\tilde{R}_{rs}}$.

With returning reference to FIGURE 2, in the case of the $R_{rs}$ estimator **134** only the $R_{rs}(t)$ estimate, $\tilde{R}_{rs}(t)$, output by the MLS operation **218** is retained while the other estimation outputs (e.g. $P_{mus}(t)$ polynomial coefficients) are discarded. In the final stage of the $R_{rs}$ estimator **134**, a Kalman filter **220** is applied to further improve the $R_{rs}$ estimation output by the MWLS **218**. The Kalman filter **220** is suitably similar to the Kalman filter **212** described above with respect to the $E_{rs}$ estimator **132**. The Kalman filter **220** for the $R_{rs}$ estimator **134** can be designed to reduce the MWLS estimation noise based on the following assumptions: (1) a state process equation where $R_{rs}$ changes slowly and can be modelled as a random walk, i.e. $R_{rs}(t) = R_{rs}(t - 1) + \omega_R(t)$, $\omega_R(t- 1) \sim N(0, \delta_R)$ ; and (2) an observation equation where the MWLS estimate $\tilde{R}_{rs}(t)$ can be modelled as $\tilde{R}_{rs}(t) = R_{rs}(t) + \omega_{LR}(t)$, where $\omega_{LR}(t) \sim N(0, \delta_{\tilde{R}_{rs}}(t))$. A standard Kalman filter can be implemented with $A = 1$, $B = 0$, $Q = \delta_R$, $H = 1$, and $R = \delta_{\tilde{R}_{rs}}(t)$. Again, the Kalman filter has certain advantages, including computationally efficient implementation in the context of a sliding time window, intuitive operation and output of weighted averages. The parameter $\delta_R$ is an algorithm parameter that controls the average window length.

The output **222** of the $R_{rs}(t)$ Kalman filter **220** is the $R_{rs}$ estimate notated here as $\tilde{R}_{rs}(t) = R_{rs}(t) + \omega_r(t)$. This output assumes that $\tilde{R}_{rs}(t)$ is an unbiased estimate of $R_{rs}(t)$, but has a noise term $\omega_r(t) \sim N(0, \delta_{\tilde{R}_{rs}})$.

With reference back to FIGURE 2, in the final pass, once the $E_{rs}$ and $R_{rs}$ estimates are obtained by the respective estimators **132, 134,** the $P_{mus}(t)$ estimator **136** is applied to estimate $P_{mus}(t)$. Using the previously estimated $R_{rs}(t)$ and $C_{rs}(t)$, a $P_{mus}(t)$ computation **224** computes the $\tilde{P}_{mus}(t)$ estimate according to:

$$\tilde{P}_{mus}(t) = P_y(t) - \hat{E}_{rs}(t)V(t) - \hat{R}_{rs}(t)\dot{V}(t)$$

evaluated over the samples of $P_y(t)$, $\dot{V}(t)$, and (via integrator **122**) $V(t)$ in the time window **130.** Said another way, $\tilde{P}_{mus}(t)$ = $P_y(t) - \hat{R}_{rs}\dot{V}(t) - \hat{E}_{rs}V(t)$ is evaluated in the time window **130** of the MWLS. To remove high-frequency noise in $\tilde{P}_{mus}(t)$, an optional lowpass filter **226** can be used to further improve the $P_{mus}(t)$ estimate. Additionally or alternatively, physiological knowledge of the $P_{mus}(t)$ waveform can be infused to further improve the $P_{mus}(t)$ estimation.

In the illustrative embodiments, the respiratory elastance (or compliance) estimator **132** is applied first, followed by the respiratory resistance estimator **134** and finally the respiratory muscle pressure estimator **136.** However, it is contemplated to estimate the respiratory resistance first, followed by estimation of the respiratory elastance or compliance (that is, to reverse the order of the estimators **132, 134**). In such a variant embodiment, the second ($E_{rs}$) estimator would suitably include an $R_{rs}$ cancellation operation analogous to the operation **216** of the illustrative embodiment. Regardless of the order of estimation of $E_{rs}$ (or $C_{rs}$) and $R_{rs}$, it will be appreciated that the final $P_{mus}(t)$ estimator **136** could optionally be omitted if $P_{mus}(t)$ and WoB (computed therefrom by integrator **140**) are not used.

If the respiratory elastance (or compliance) and/or resistance are displayed on the display component **110** of the ventilator **100,** these values may optionally also be displayed with their respective uncertainty metrics, for example expressed in terms of the $\delta$ or $\omega$ statistics described herein or functions thereof. While these or other respiratory parameters are described as being displayed on the display component **110** of the ventilator **100** in the illustrative examples, it will be appreciated that such values may additionally or alternatively be displayed on a bedside patient monitor, at a nurses' station computer, and/or may be stored in an Electronic Health Record (EHR) or other patient data storage system, or so forth. The illustrative respiratory system analyzer **120** is suitably implemented via the microprocessor of the ventilator **100;** however, the respiratory system analyzer **120** could additionally or alternatively be implemented via a microprocessor of a bedside patient monitor or other electronic data processing device. The disclosed respiratory system analyzer functionality may also be embodied by a non-transitory storage medium storing instructions that are readable and executable by such a microprocessor or other electronic data processing device to perform the disclosed functionality. By way of example, the non-transitory storage medium may, for example, include a hard disk or other magnetic storage medium, optical disk or other optical storage medium, flash memory or other electronic storage medium, various combinations thereof, or so forth.

As previously noted, in addition to displaying one or more of the estimated values (e.g. one or more of the values $\hat{E}_{rs}(t)$, $\hat{C}_{rs}(t) = 1/\hat{E}_{rs}$, $\hat{R}_{rs}(t)$, $\hat{P}_{mus}(t)$ optionally with its statistical uncertainty) as a real-time value, trend line or so forth, in another illustrative application the $\hat{P}_{mus}(t)$ waveform may be used to synchronize the positive pressure applied by the ventilator **100** with respiratory effort expended by the patient **102,** so as to reduce patient-ventilator dyssynchrony. In this application, the positive air pressure applied by the ventilator **100** is adjusted, e.g. increased or decreased, in synch with increasing or decreasing magnitude of $\hat{P}_{mus}(t)$. In another control application, the WoB output by the integrator **140** may be used as a feedback signal for control of the ventilator **100**. In general, the positive pressure applied by the ventilator **100** should increase with increasing measured WoB output by integrator **140,** and this increased mechanical ventilation should result in a consequent reduction in patient WoB until the setpoint WoB is reached. As illustration, a proportional and/or derivative and/or integral controller (e.g. PID controller) may be used for this feedback control with the WoB signal from the integrator **140** serving as the feedback signal, a target WoB serving as the setpoint value, and the positive pressure being the controlled variable.

The respiratory system analyzer **120** has been tested with simulated data and with pig respiratory data, and the results show the analyser **120** can provide comparable results to invasive solutions and is stable under different ventilator settings, including low PSV settings. The analyzer **120** provides various benefits, including (but not limited to): providing real-time data (with a lag of a few seconds or less); sample-by-sample estimation (if successive windows overlap and are spaced by a single sample); tailorable trade-off between computational complexity and temporal resolution (faster computation by larger spacing between possibly overlapping windows traded off against reduced temporal resolution); rapid convergence (within 10 breaths in some tests) providing low initiation time; stability against unexpected disturbances; good computational efficiency employing, for example, efficient pseudo-inverse (L$\times$4) matrix computation (where L is the window size, e.g. 60-90 samples in some suitable embodiments); and low memory requirements (storing the data for the current time window, around 60-90 samples for some embodiments).

As a further advantage, the respiratory system analyzer **120** suitably estimates the elastance or compliance $E_{rs}(t)$, resistance $R_{rs}(t)$, and respiratory muscle pressure $P_{mus}(t)$ without receiving as input the respiratory phase or respiratory rate, and without making any *a priori* assumptions about these parameters (other than that $E_{rs}$ and $R_{rs}$ are assumed to be constant within any given time window of the MWLS estimation). The respiratory system analyzer **120** suitably operates only on the measured air pressure $P_y(t)$ and air flow $\dot{V}(t)$ along with $V(t) = \int\dot{V}(t)dt$ which is derived by integrating $\dot{V}(t)$ over time.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims or the equivalents thereof.

**Claims**

1. A medical ventilator device comprising:

   a ventilator (100) configured to deliver ventilation to a ventilated patient (102);
   a pressure sensor (112) configured to measure the airway pressure $P_y(t)$ of the ventilated patient; and
   an air flow sensor (114) configured to measure the air flow $\dot{V}(t)$ into and out of the ventilated patient, and **characterised by** further comprising
   a respiratory system analyzer (120) comprising a microprocessor configured to use a moving time window (130) least squares (MWLS) estimation applied to the measured airway pressure $P_y(t)$ of the ventilated patient and the measured air flow $\dot{V}(t)$ into and out of the ventilated patient by evaluating the Equation of Motion of the Lungs $P_y(t) = R_{rs}\dot{V}(t) + E_{rs}V(t) + P_{mus}(t)$ to estimate respiratory parameters of the ventilated patient including (i) respiratory system elastance or compliance ($E_{rs}$ or $C_{rs}$), (ii) respiratory system resistance ($R_{rs}$), and (iii) respiratory muscle pressure ($P_{mus}(t)$), wherein the duration of the time window (130) is a fraction of the breathing cycle duration so that the parameters $E_{rs}$ and $R_{rs}$ can be assumed to be constant within each time window (130), and wherein $P_{mus}(t)$ within each time window (130) is approximated by using a low-order polynomial function.

2. The medical ventilator device of claim 1 wherein the MWLS estimation includes, for each time window of the MWLS estimation, performing the following operations in order:

   (1) estimating one of (i) elastance or compliance and (ii) resistance;
   (2) estimating the other of (i) elastance or compliance and (ii) resistance using the estimated value from operation (1); and
   (3) estimating respiratory muscle pressure using the values estimated in operations (1) and (2).

3. The medical ventilator device of claim 2 wherein the operation (1) estimates elastance or compliance and the operation (2) estimates resistance using the estimated value of elastance or compliance from operation (1).

4. The medical ventilator device of any one of claims 2-3 wherein the operation (1) optimizes elastance $E_{rs}$, resistance $R_{rs}$, and the difference $\Delta P_{mus}$ of the respiratory muscle pressure $P_{mus}$ of the equation:

$$\Delta P_y(t) = R_{rs}\Delta\dot{V}(t) + E_{rs}\Delta V(t) + \Delta P_{mus}$$

   with respect to the measured values of $P_y(t)$ and $\dot{V}(t)$ in the time window of the MWLS where $V(t) = \int\dot{V}(t)dt$ and $\Delta P_y(t) = P_y(t) - P_y(t-1)$ and $\Delta\dot{V}(t) = \dot{V}(t) - \dot{V}(t-1)$ and $\Delta V(t) = V(t) - V(t-1)$.

5. The medical ventilator device of claim 4 wherein the operation (2) optimizes the respiratory muscle pressure $P_{mus}(t)$ and one of elastance $E_{rs}$ and resistance $R_{rs}$ of the equation:

$$P_y(t) = R_{rs}\dot{V}(t) + E_{rs}V(t) + P_{mus}(t)$$

   with respect to the measured values of $P_y(t)$ and $\dot{V}(t)$ in the time window of the MWLS with the estimated value from operation (1) held fixed and $P_{mus}(t)$ modeled by a parameterized function of time.

6. The medical ventilator device of claim 5 wherein operation (2) is repeated with $P_{mus}(t)$ modeled by zeroeth, first, and second order polynomial functions of time and the optimized elastance $E_{rs}$ or resistance $R_{rs}$ of the three repetitions are combined.

7. The medical ventilator device of any one of claims 5-6 wherein the operation (3) estimates respiratory muscle pressure as $P_y(t) - \hat{R}_{rs}\dot{V}(t) - \hat{E}_{rs}V(t)$ in the time window of the MWLS where $\hat{R}_{rs}$ and $\hat{E}_{rs}$ are estimated values from operations (1) and (2).

8. The medical ventilator device of any one of claims 2-7 wherein one or both of the operations (1) and (2) includes applying a Kalman filter (212, 220) to the estimated value.

9. The medical ventilator device of claim 8 wherein one or both of the operations (1) and (2) further includes generating an uncertainty metric for the estimated value based on a noise variance of the Kalman filter (212, 220).

10. The medical ventilator device of any one of claims 1-9 further comprising:
   a display (110) configured to display one or more of the respiratory parameters of the ventilated patient estimated by the respiratory system analyzer.

11. The medical ventilator device of any one of claims 1-10 wherein the ventilator (100) is programmed to adjust positive air pressure output by the ventilator in synch with increasing or decreasing magnitude of the respiratory muscle pressure ($P_{mus}(t)$) in order to reduce patient-ventilator dyssynchrony.

12. The medical ventilator device of any one of claims 1-11 wherein:

   the respiratory system analyzer (120) is configured to estimate a work of breathing (WoB) as $WoB = \int P_{mus}(t)dV(t)$ where $P_{mus}(t)$ is the respiratory muscle pressure as a function of time estimated using the MWLS estimation; and
   the ventilator (100) is programmed to control mechanical ventilation provided by the ventilator to maintain the estimated WoB at a setpoint WoB value.

13. A method comprising:
   measuring, during ventilation of a patient, airway pressure $P_y(t)$ and air flow $\dot{V}(t)$ of air into and out of the patient, and **characterised by** further comprising:
   using a microprocessor for applying a moving time window (130) least squares (MWLS) estimation to the measured airway pressure $P_y(t)$ and the measured air flow $\dot{V}(t)$ into and out of the patient by evaluating the Equation of Motion of the Lungs $P_y(t) = R_{rs}\dot{V}(t) + E_{rs}V(t) + P_{mus}(t)$ to estimate respiratory parameters of the patient including (i) respiratory system elastance or compliance ($E_{rs}$ or $C_{rs}$), (ii) respiratory system resistance ($R_{rs}$), and (iii) respiratory muscle pressure ($P_{mus}(t)$), wherein the duration of the time window (130) is a fraction of the breathing cycle duration so that the parameters $E_{rs}$ and $R_{rs}$ can be assumed to be constant within each time window (130), and wherein $P_{mus}(t)$ within each time window (130) is approximated by using a low-order polynomial function.

14. A non-transitory storage medium storing instructions readable and executable by an electronic data processing device to perform a method according to claim 13.

**Patentansprüche**

1. Ein Beatmungsgerät, das Folgendes umfasst:

   ein Beatmungsgerät (100), das den zu beatmenden Patienten (102) beatmet;
   einen Drucksensor (112), mit dem der Atemwegsdruck $P_y(t)$ des zu beatmenden Patienten gemessen wird; und
   einen Luftmengensensor (114), mit dem die Luftmenge $\dot{V}(t)$ von und zum zu beatmenden Patienten gemessen wird; und zusätzlich
   einem Atmungssystem-Analysegerät (120), das aus einem Mikroprozessor besteht, der die Schätzung der kleinsten Quadrate für ein veränderliches Zeitfenster (130) (VZKQ) für den gemessenen Atemwegsdruck $P_y(t)$ des zu beatmenden Patienten sowie die gemessene Luftmenge $\tilde{V}(t)$ von und zum zu beatmenden Patienten übernimmt, indem die Bewegungsgleichung der Lungen $P_y(t) = R_{rs}\tilde{V}(t) + E_{rs}V(t) + P_{mus}(t)$ ausgewertet wird, um die Atmungsparameter des zu beatmenden Patienten einschließlich (i) der Elastanz oder Übereinstimmung des Atmungssystems ($E_{rs}$ oder $C_{rs}$), (ii) der Widerstandsfähigkeit des Atmungssystems ($R_{rs}$) und (iii) des Atemwegsmuskeldrucks ($P_{mus}(t)$), wobei die Dauer des Zeitfensters (130) einem Bruchteil der Dauer des Atmungszyklus entspricht, sodass davon ausgegangen werden kann, dass die Parameter $E_{rs}$ und $R_{rs}$ innerhalb der einzelnen Zeitfenster (130) konstant sind. Zudem wird $P_{mus}(t)$ für die einzelnen Zeitfenster (130) mithilfe einer Polynomfunktion niedriger Ordnung angeglichen.

2. Das Beatmungsgerät gemäß Anspruch 1, wobei für die VZKQ-Schätzung in den einzelnen Zeitfenstern der VZKQ-Schätzung folgende Schritte in der angegebenen Reihenfolge durchgeführt werden:

   (1) Schätzen entweder (i) der Elastanz oder der Übereinstimmung sowie (ii) der Widerstandsfähigkeit;
   (2) Schätzen der bislang übergangenen (i) Elastanz oder Übereinstimmung sowie (ii) der Widerstandsfähigkeit anhand des Schätzwerts von Schritt (1); und

(3) Schätzen des Atemwegsmuskeldrucks anhand der Schätzwerte der Schritte (1) und (2).

3. Das Beatmungsgerät gemäß Anspruch 2, wobei in Schritt (1) die Elastanz oder Übereinstimmung und in Schritt (2) die Widerstandsfähigkeit anhand des Schätzwerts für die Elastanz oder Übereinstimmung von Schritt (1) geschätzt wird.

4. Das Beatmungsgerät gemäß einer der Ansprüche 2 oder 3, wobei in Schritt (1) die Elastanz $E_{rs}$, die Widerstandsfähigkeit $R_{rs}$ und die Differenz $\Delta P_{mus}$ des Atemwegsmuskeldrucks $P_{mus}$ der folgenden Gleichung optimiert wird:

$$\Delta P_y(t) = R_{rs}\Delta \tilde{V}(t) + E_{rs}\Delta V(t) + \Delta P_{mus}$$

Dies erfolgt unter Berücksichtigung der gemessenen Werte für $P_y(t)$ und $\tilde{V}(t)$ im Zeitfenster des VZKQ, wobei gilt: $V(t) = \int \tilde{V}(t)dt$ und $\Delta P_Y(t) = P_y(t) - P_y(t-1)$ und $\Delta \tilde{V}(t) = \tilde{V}(t) - \tilde{V}(t-1)$ und $\Delta V(t) = V(t) - V(t-1)$.

5. Das Beatmungsgerät gemäß Anspruch 4, wobei in Schritt (2) der Atemwegsmuskeldruck $P_{mus}(t)$ sowie entweder die Elastanz $E_{rs}$ oder die Widerstandsfähigkeit $R_{rs}$ der folgenden Gleichung optimiert wird:

$$P_y(t) = R_{rs}\tilde{V}(t) + E_{rs}V(t) + P_{mus}(t)$$

Hierbei werden die Messwerte für $P_y(t)$ und $\tilde{V}(t)$ für das Zeitfenster des VZKQ berücksichtigt, während der Schätzwert von Schritt (1) beibehalten und $P_{mus}(t)$ anhand einer parametrisierten Zeitfunktion modelliert wird.

6. Das Beatmungsgerät gemäß Anspruch 5, wobei Schritt (2) wiederholt wird. Hierzu wird $P_{mus}(t)$ herangezogen, das anhand einer Polynomfunktion der nullten, ersten oder zweiten Ordnung modelliert wird. Zudem werden die optimierte Elastanz $E_{rs}$ oder die Widerstandsfähigkeit $R_{rs}$ der drei Wiederholungen kombiniert.

7. Das Beatmungsgerät gemäß einer der Ansprüche 5 oder 6, wobei in Schritt (3) der Atemwegsmuskeldruck als

$P_y(t) - \hat{R}_{rs}\tilde{V}(t) - \hat{E}_{rs}V(t)$ im Zeitfenster des VZKQ geschätzt wird, wobei es sich bei $\hat{R}_{rs}$ und $\hat{E}_{rs}$ um die Schätzwerte der Schritte (1) und (2) handelt.

8. Das Beatmungsgerät gemäß einer der Ansprüche 2 bis 7, wobei in mindestens einem der Schritte (1) und (2) dem Schätzwert ein Kalman-Filter (212, 220) hinzugefügt wird.

9. Das Beatmungsgerät gemäß Ansprüche 8, wobei in mindestens einem der Schritte (1) und (2) zudem eine Unsicherheitsmetrik für den Schätzwert generiert wird, die auf der Varianz des Rauschens des Kalman-Filters (212, 220) beruht.

10. Das Beatmungsgerät gemäß einer der Ansprüche 1 bis 9, wobei dieses zudem Folgendes umfasst:
eine Anzeige (110), auf der mindestens einer der Atmungsparameter des zu beatmenden Patienten angezeigt wird, der mithilfe des Atmungssystem-Analysegeräts geschätzt wird.

11. Das Beatmungsgerät gemäß einer der Ansprüche 1 bis 10, wobei das Beatmungsgerät (100) so programmiert ist, dass die positive Luftdruckabgabe des Beatmungsgeräts mit dem zu- oder abnehmenden Atemwegsmuskeldruck ($P_{mus}(t)$) synchronisiert ist, um die Ungleichzeitigkeit von Patient und Beatmungsgerät zu verringern.

12. Das Beatmungsgerät gemäß einer der Ansprüche 1 bis 11, wobei:

das Atmungssystem-Analysegerät (120) die Atemarbeit (WoB) als $WoB = \int P_{mus}(t)dV(t)$ schätzt, wobei $P_{mus}(t)$ dem Atemwegsmuskeldruck als mit der VZKQ-Schätzung geschätzte Zeitfunktion entspricht; und
das Beatmungsgerät (100) so programmiert ist, dass es die mechanische Beatmung des Beatmungsgeräts steuert, um die geschätzte WoB auf einem WoB-Sollwert zu halten.

13. Eine Methode, die Folgendes umfasst:
Messen des Atemwegsdrucks $P_y(t)$ und der Luftmenge $\tilde{V}(t)$ von und zum zu beatmenden Patienten während der Beatmung, sowie zudem:

Verwenden eines Mikroprozessors, der die Schätzung der kleinsten Quadrate für ein veränderliches Zeitfenster (130) (VZKQ) für den gemessenen Atemwegsdruck $P_y(t)$ sowie die gemessene Luftmenge $\tilde{V}(t)$ von und zum Patienten übernimmt, indem die Bewegungsgleichung der Lungen $P_y(t) = R_{rs} \tilde{V}(t) + E_{rs}V(t) + P_{mus}(t)$ ausgewertet wird, um die Atmungsparameter des Patienten einschließlich (i) der Elastanz oder Übereinstimmung des Atmungssystems ($E_{rs}$ oder $C_{rs}$), (ii) der Widerstandsfähigkeit des Atmungssystems ($R_{rs}$) und (iii) des Atemwegsmuskeldrucks ($P_{mus}(t)$), wobei die Dauer des Zeitfensters (130) einem Bruchteil der Dauer des Atmungszyklus entspricht, sodass davon ausgegangen werden kann, dass die Parameter $E_{rs}$ und $R_{rs}$ innerhalb der einzelnen Zeitfenster (130) konstant sind. Zudem wird $P_{mus}(t)$ für die einzelnen Zeitfenster (130) mithilfe einer Polynomfunktion niedriger Ordnung angeglichen.

14. Ein nicht flüchtiges Speichermedium, auf dem Anweisungen gespeichert werden, die von einem elektronischen Datenverarbeitungsgerät gelesen und ausgeführt werden können, um die Methode gemäß Anspruch 13 umzusetzen.

**Revendications**

1. Dispositif de ventilation médical comprenant :

   un ventilateur (100) conçu pour fournir une ventilation à un patient ventilé (102) ;
   un capteur de pression (112) conçu pour mesurer la pression des voies aériennes $P_y(t)$ du patient ventilé ; et
   un capteur de débit d'air (114) conçu pour mesurer un débit d'air $\tilde{V}(t)$ entrant et sortant du patient ventilé, et **caractérisé en ce que** ledit dispositif comprend en outre
   un analyseur de système respiratoire (120) comprenant un microprocesseur configuré pour utiliser une estimation des moindres carrés d'une fenêtre temporelle (130) mobile (MWLS) appliquée à la pression des voies aériennes $P_y(t)$ mesurée du patient ventilé et le débit d'air $\tilde{V}(t)$ mesuré entrant et sortant du patient ventilé par évaluation de l'équation du mouvement des poumons $P_y(t) = R_{rs}\tilde{V}(t) + E_{rs}\tilde{V}(t) + P_{mus}(t)$ pour estimer les paramètres respiratoires du patient ventilé, en particulier (i) une élastance ou une compliance du système respiratoire ($E_{rs}$ ou $C_{rs}$), (ii) une résistance du système respiratoire ($R_{rs}$), et (iii) une pression musculaire respiratoire ($P_{mus}(t)$), dans lequel la durée de la fenêtre temporelle (130) est une fraction de la durée du cycle respiratoire de telle sorte que les paramètres $E_{rs}$ et $R_{rs}$ peuvent être supposés constants dans chaque fenêtre temporelle (130), et dans lequel $P_{mus}(t)$ dans chaque fenêtre temporelle (130) est approximé à l'aide d'une fonction polynomiale d'ordre faible.

2. Dispositif de ventilation médical selon la revendication 1, dans lequel l'estimation MWLS comprend, pour chaque fenêtre temporelle de l'estimation MWLS, l'exécution des opérations suivantes dans l'ordre :

   (1) l'estimation (i) de l'élastance ou de la compliance, et (ii) de la résistance ;
   (2) l'estimation de l'autre parmi (i) l'élastance ou la compliance, et (ii) de la résistance à l'aide de la valeur estimée de l'opération (1) ; et
   (3) l'estimation de la pression musculaire respiratoire à l'aide des valeurs estimées dans les opérations (1) et (2).

3. Dispositif de ventilation médical selon la revendication 2, dans lequel l'opération (1) estime l'élastance ou la compliance et l'opération (2) estime la résistance à l'aide de la valeur estimée de l'élastance ou de la compliance de l'opération (1).

4. Dispositif de ventilation médical selon l'une quelconque des revendications 2 à 3, dans lequel l'opération (1) optimise l'élastance $E_{rs}$, la compliance $R_{rs}$, et la différence $\Delta P_{mus}$ de la pression musculaire respiratoire $P_{mus}$ de l'équation :

$$\Delta P_y(t) = R_{rs}\Delta \tilde{V}(t) + E_{rs}\Delta V(t) + \Delta P_{mus}$$

par rapport aux valeurs mesurées de $P_y(t)$ et $\tilde{V}(t)$ dans la fenêtre temporelle de la MWLS où $V(t) = \tilde{V}(t)dt$ et $\Delta P_y(t) = P_y(t) - P_y(t - 1)$ et $\Delta \tilde{V}(t) = \tilde{V}(t) - \tilde{V}(t - 1)$ et $\Delta V(t) = V(t) - V(t - 1)$ .

5. Dispositif de ventilation médical selon la revendication 4, dans lequel l'opération (2) optimise la pression musculaire respiratoire $P_{mus}(t)$ et l'élastance $E_{rs}$ ou la résistance $R_{rs}$ de l'équation :

$$P_y(t) = R_{rs}\tilde{V}(t) + E_{rs}V(t) + P_{mus}(t)$$

par rapport aux valeurs mesurées de $P_y(t)$ et $\tilde{V}(t)$ dans la fenêtre temporelle de la MWLS avec la valeur estimée de l'opération (1) maintenue fixe et la $P_{mus}(t)$ modélisée par une fonction paramétrée du temps.

6. Dispositif de ventilation médical selon la revendication 5, dans lequel l'opération (2) est répétée avec la $P_{mus}(t)$ modélisée par des fonctions polynomiales de temps zéro, de premier et de second ordres et l'élastance optimisée $E_{rs}$ ou la résistance $R_{rs}$ des trois répétitions sont combinées.

7. Dispositif de ventilation médical selon l'une quelconque des revendications 5 à 6, dans lequel l'opération (3) estime la pression musculaire respiratoire en tant que $P_y(t) - \hat{R}_{rs}\tilde{V}(t) - \hat{E}_{rs}V(t)$ dans la fenêtre temporelle de la MWLS où $R_{rs}$ et $\hat{E}_{rs}$ sont des valeurs estimées des opérations (1) et (2).

8. Dispositif de ventilation médical selon l'une quelconque des revendications 2 à 7, dans lequel l'une des opérations (1) et (2) ou les deux comprennent l'application d'un filtre de Kalman (212, 220) à la valeur estimée.

9. Dispositif de ventilation médical selon la revendication 8, dans lequel l'une des opérations (1) et (2) ou les deux comprennent en outre la génération d'une métrique d'incertitude pour la valeur estimée en fonction d'une variance de bruit du filtre de Kalman (212, 220).

10. Dispositif de ventilation médical selon l'une quelconque des revendications 1 à 9, comprenant en outre :
un affichage (110) conçu pour afficher au moins un paramètre respiratoire du patient ventilé estimé par l'analyseur du système respiratoire.

11. Dispositif de ventilation médical selon l'une quelconque des revendications 1 à 10, dans lequel le ventilateur (100) est programmé pour régler la sortie de pression d'air positive par le ventilateur en synchronisation avec l'augmentation ou la diminution de l'amplitude de la pression musculaire respiratoire ($P_{mus}(t)$) pour réduire la dyssynchronie patient-ventilateur.

12. Dispositif de ventilation médical selon l'une quelconque des revendications 1 à 11, dans lequel :

l'analyseur de système respiratoire (120) est conçu pour estimer un travail de respiration (WoB) en tant que $WoB = \int P_{mus}(t)dV(t)$ où $P_{mus}(t)$ est la pression musculaire respiratoire en tant que fonction du temps estimé à l'aide de l'estimation MWLS ; et
le ventilateur (100) est programmé pour commander la ventilation mécanique fournie par le ventilateur pour maintenir le WoB estimé à une valeur de WoB de consigne.

13. Procédé comprenant :
la mesure, lors de la ventilation d'un patient, de la pression des voies aériennes $P_y(t)$ et du débit d'air $\tilde{V}(t)$ d'air entrant et sortant du patient, et **caractérisé en ce que** ledit procédé comprend en outre :
l'utilisation d'un microprocesseur pour appliquer une estimation des moindres carrés d'une fenêtre temporelle (130) mobile (MWLS) à la pression des voies aériennes $P_y(t)$ mesurée et au débit d'air $\tilde{V}(t)$ mesuré entrant et sortant du patient par évaluation de l'équation du mouvement des poumons $P_y(t) = R_{rs}\tilde{V}(t) + E_{rs}V(t) + P_{mus}(t)$ pour estimer les paramètres respiratoires du patient, y compris (i) l'élastance ou la compliance du système respiratoire ($E_{rs}$ ou $C_{rs}$), (ii) la résistance du système respiratoire ($R_{rs}$) et (iii) la pression musculaire respiratoire ($P_{mus}(t)$), dans lequel la durée de la fenêtre temporelle (130) est une fraction de la durée du cycle respiratoire de telle sorte que les paramètres $E_{rs}$ et $R_{rs}$ peuvent être supposés constants dans chaque fenêtre temporelle (130), et dans lequel $P_{mus}(t)$ dans chaque fenêtre temporelle (130) est approximé à l'aide d'une fonction polynomiale d'ordre faible.

14. Support d'enregistrement non transitoire mémorisant des instructions lisibles et exécutables par un dispositif électronique de traitement des données pour mettre en œuvre un procédé selon la revendication 13.

FIG. 1

EP 3 302 664 B1

FIG. 2

MWLS: Moving Window Least Square, $E_{rs}(t) = 1/C_{rs}(t)$

$130_{n+1}$

$130_n$

moving window

L: LS window size

$P_{mus}$ (cmH$_2$O)

Time (s)

$[\tilde{E}_{rs}(t), \tilde{R}_{rs}(t), \Delta\tilde{P}_{mus}(t)]^T, \delta_{E_{rs}}(t)$
(over the window (t-L+1,t))

$[\tilde{E}_{rs}(t+1), \tilde{R}_{rs}(t+1), \Delta\tilde{P}_{mus}(t+1)]^T, \delta_{E_{rs}}(t+1)$
(over the window (t-L+2,t+1))

FIG. 3

EP 3 302 664 B1

FIG. 4

EP 3 302 664 B1

$$\tilde{R}_{rs} = \tilde{R}_{rs,0} * w_0 + \tilde{R}_{rs,1} * w_1 + \tilde{R}_{rs,2} * w_2$$

$$w_0 = \frac{\dfrac{1}{\delta\tilde{R}_{rs},0}}{\dfrac{1}{\delta\tilde{R}_{rs},0} + \dfrac{1}{\delta\tilde{R}_{rs},1} + \dfrac{1}{\delta\tilde{R}_{rs},2}}$$

$$w_1 = \frac{\dfrac{1}{\delta\tilde{R}_{rs},1}}{\dfrac{1}{\delta\tilde{R}_{rs},0} + \dfrac{1}{\delta\tilde{R}_{rs},1} + \dfrac{1}{\delta\tilde{R}_{rs},2}}$$

$$w_2 = \frac{\dfrac{1}{\delta\tilde{R}_{rs},2}}{\dfrac{1}{\delta\tilde{R}_{rs},0} + \dfrac{1}{\delta\tilde{R}_{rs},1} + \dfrac{1}{\delta\tilde{R}_{rs},2}}$$

$$\delta\tilde{R}_{rs} = \frac{1}{\dfrac{1}{\delta\tilde{R}_{rs},0} + \dfrac{1}{\delta\tilde{R}_{rs},1} + \dfrac{1}{\delta\tilde{R}_{rs},2}}$$

218

$218_0$ — MWLS with 0-order Pmus — $\tilde{R}_{rs,0}$

$218_1$ — MWLS with 1-order Pmus — $\tilde{R}_{rs,1}$

$218_2$ — MWLS with 2-order Pmus — $\tilde{R}_{rs,2}$

219 — Maximum Ratio Combination — $\tilde{R}_{rs}$

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030010339 A1
- US 20040040560 A1
- EP 0904730 A1
- WO 2014207623 A2